Europäisches Patentamt

European Patent Office

Office européen des brevets

(11) Numéro de publication: **0 451 233 B1**

# (12) FASCICULE DE BREVET EUROPEEN

(45) Date de publication de fascicule du brevet: **01.03.95**  (51) Int. Cl.⁶: **A61B  5/00**

(21) Numéro de dépôt: **90914732.4**

(22) Date de dépôt: **05.10.90**

(86) Numéro de dépôt internationale :
**PCT/EP90/01678**

(87) Numéro de publication internationale :
**WO 91/04704 (18.04.91 91/09)**

(54) **DISPOSITIF IMPLANTABLE D'EVALUATION DU TAUX DE GLUCOSE.**

(30) Priorité: **06.10.89 FR 8913069**

(43) Date de publication de la demande:
**16.10.91 Bulletin  91/42**

(45) Mention de la délivrance du brevet:
**01.03.95 Bulletin  95/09**

(84) Etats contractants désignés:
**AT BE CH DE DK ES FR GB IT LI LU NL SE**

(56) Documents cités:
**EP-A- 0 245 075**
**WO-A-81/01794**
**US-A- 4 538 616**
**US-A- 4 822 336**

**Sensors and Actuators, A21-A23, 1990, pages 58-61; Ylva Bäcklund et al.; "Passive Silicon Transenor Intended for Biomedical, Remote Pressure Monitoring"**

(73) Titulaire: **NEFTEL, Frédéric**
**1 rue des Ecouffes**
**F-75004 Paris (FR)**

(72) Inventeur: **NEFTEL, Frédéric**
**1 rue des Ecouffes**
**F-75004 Paris (FR)**

(74) Mandataire: **Léger, Jean-François**
**Micheli & Cie**
**Groupement 101**
**122, rue de Genève**
**Case postale 61**
**CH-1226 Genève-Thônex (CH)**

## Description

La présente invention se rapporte au domaine des dispositifs médicaux et, plus particulièrement, à un dispositif implantable permettant une évaluation précise et simple du taux de glucose sanguin d'un individu.

On connait aujourd'hui les problèmes liés à la détermination du taux de glucose sanguin chez le diabétique, notamment par l'aspect contraignant et agressif des méthodes utilisées. Il peut s'agir, par exemple, de prélever 2 à 3 fois par jour une goutte de sang à l'extrémité d'un doigt que l'on place sur un "glucomètre" et qui donne un résultat précis très rapidement mais qui, à terme, laisse des séquelles importantes. Il peut également s'agir d'une électrode sous-cutanée, reliée à l'extérieur par des connections électriques, qui peut rester 3 à 7 jours en place mais qui nécessite des précautions importantes d'asepsie et représente une gêne permanente pour le patient.

Pourtant, une mesure précise du taux de glucose sanguin est indispensable à un bon équilibrage des doses d'insuline à administrer à chaque repas. A défaut, le malade s'expose à des risques importants. Parmi les risques à court terme figurent l'hypoglycémie ou l'hyperglycémie pouvant aller jusqu'au coma et, plus encore parmi les risques à long terme, tous les problèmes d'artériopathie périphérique (artériosclérose oblitérante) pouvant conduire à la cécité ou à une amputation des extrémités. Confrontés à ces problèmes graves, les diabétiques ont appris à ajuster les doses d'insuline par des mesures répétées du taux de glucose et à établir un rythme alimentaire et physique strict. C'est dire l'importance et la répercussion pour ces malades des conditions d'évaluation du taux de glucose sanguin.

L'invention a pour but de permettre une évaluation précise du taux de glucose sanguin d'un individu, par une méthode non agressive et à travers un dispositif de très faible encombrement qui est implanté, par exemple, en territoire sous-cutané.

On sait qu'il existe une très bonne correspondance entre le taux de glucose sanguin et le taux de glucose interstitiel régnant, par exemple, dans le territoire sous-cutané. En effet, le glucose diffuse librement à travers les vaisseaux jusque dans le territoire interstitiel.

On sait également que les modifications essentielles de composition du liquide interstitiel sont liées à une modification d'un nombre limité de substances, dont le glucose. Parmi ces substances et pour un encombrement spatial de l'ordre de celui de la molécule de glucose (environ 0,8nm), c'est le glucose qui varie de façon la plus significative. C'est pourquoi, une mesure sélective de la pression osmotique dûe aux molécules ayant un encombrement spatial équivalent à celui du glucose reflète-t-il, de façon assez précise, la pression osmotique du glucose dans ce territoire et, par conséquent, le taux de glucose sanguin. Ce type de mesure peut naturellement s'effectuer dans n'importe quel compartiment en équilibre glucosique avec le compartiment sanguin (par exemple le territoire sous-cutané ou la cavité péritonéale).

Certains auteurs ont pu prétendre à la détermination du taux de glucose par la seule mesure de la pression osmotique exercée par l'ensemble des molécules ayant un encombrement spatial supérieur à celui du glucose, une telle mesure étant effectuée à l'aide d'une membrane hémiperméable au glucose. On peut noter, en particulier, le brevet américain N° 4.538.616 de Robert Rogoff qui décrit un tel système. Néanmoins, il convient de noter que des variations de l'hydratation du patient, ou plus encore de certains acides aminés, pour ne prendre que quelques exemples, peuvent être la cause d'une variation importante de la pression osmotique de l'individu et, dans l'approche décrite, conduire à une erreur d'interprétation dont les conséquences peuvent être extrêmement graves pour le patient.

Le principe, selon l'invention, consiste à mesurer de façon précise, dans un territoire interstitiel, la valeur absolue - ou les variations - de la pression osmotique dûe aux molécules ayant un encombrement spatial équivalent à celui du glucose à l'aide d'un dispositif implanté de très faible encombrement. Une telle mesure pouvant s'effectuer dans tout territoire ou compartiment en équilibre glucosique avec le compartiment vasculaire. De la valeur mesurée, on déduit la pression osmotique dûe au glucose et, par conséquent, le taux de glucose circulant.

On connait déjà, aujourd'hui, de nombreux dispositifs miniaturisés de mesure de pression en silicium, utilisant les techniques d'usinage par photolithographie. Un capteur de pression selon de telles techniques pourrait être avantageusement utilisé étant donné son très faible encombrement final (quelques mm2 sur moins d'un mm d'épaisseur). En particulier, les caractéristiques de sensibilité obtenues sont tout à fait compatibles avec les variations de pression à mesurer qui sont de l'ordre de quelques millimètres de mercure.

On connait également différents types de membranes ayant une perforation précise permettant de laisser passer l'eau, les ions, les lactates, mais pas le glucose. De telles membranes sont dites hémiperméables au glucose. Le diamètre des perforations est, dans ce cas, compris environ entre environ 0,6nm et 0,74nm.

On connait également des membranes dont les perforations sont supérieures à la taille de la molécule de glucose et qui permettent le passage du glucose. Si on choisi une telle membrane avec des perforation ayant un diamètre de l'ordre de 0,9nm, on peut réaliser une membrane dite perméable limite au glucose.

On connait également des revêtements biocompatibles qui sont perméables au glucose et à de nombreuses molécules, mais imperméables aux cellules. De tels revêtements, tels le polymère d'acide perfluoro-sulfonique (Nafion® de Du Pont de Nemours) présentent l'avantage de ne pas s'obstruer après plusieurs années en territoire interstitiel et de ne pas être rejetés par l'organisme. Un tel revêtement peut, par conséquent, avantageusement être utilisé comme membrane de protection biocompatible.

L'invention consiste à réaliser un ensemble de deux chambres de mesures constituées par:

- d'une part une première chambre de mesure de pression osmotique directement en contact avec un premier capteur de pression et séparée du milieu interstitiel par une membrane hémiperméable au glucose, la dite membrane étant separée du milieu interstitiel par une membrane de protection biocompatible;

- d'autre part une deuxième chambre de mesure de pression osmotique directement en contact avec un deuxième capteur de pression et séparée du milieu interstitiel par une membrane perméable limite au glucose, la dite membrane étant également séparée du milieu interstitiel par une membrane de protection biocompatible.

Le dispositif selon l'invention permet de mesurer une pression osmotique absolue, ou des différences de pression osmotique, relativement aux molécules d'un encombrement équivalent à celui du glucose de façon précise. Un couplage des deux capteurs de pression à un système électronique permet de communiquer les valeurs mesurées à un récepteur situé à l'extérieur de l'individu. Un tel système électronique peut être, notamment, un système passif de type résonnant LC à fréquence variable, répondant à un signal Radio Fréquence émis depuis l'extérieur. Dans ce cas, le système électronique ne nécessite pas de source d'énergie et peut donc rester implanté à très long terme. Un tel type de détecteur de pression résonant est décrit par l'équipe suédoise de l'Institut de Technologie d'Uppsala pour un capteur dé pression intra-oculaire, présenté au congrès "Transducer 89" à Montreux en Suisse le 26 juin 1989 (Ylbva Bäcklund, Lars Rosengren et Bertril Hök de l'Institut de Technologie d'Uppsala, Björn Svedbergh de l'Institut d'Ophtalmologie d'Uppsala). Un tel système résonant est déjà couramment utilisé dans l'industrie et a été décrit pour la première fois par Collins en 1967.

D'autres buts et avantages de la présente invention apparaîtront à la lecture de la description suivante et des figures jointes, données à titre illustratif mais non limitatif.

La figure 1 est une représentation schématique de l'une des deux chambres de mesures constituant le dispositif et montrant une vue en coupe des différents éléments constituant cette chambre.

La figure 2 est une représentation schématique de l'une des deux chambres de mesures constituant le dispositif conforme à la présente invention, cette figure montrant les éléments électroniques de cette chambre et de l'émetteur externe qui lui correspond.

La figure 3 représente un type de courbe de fréquence de résonance pouvant être obtenue avec un type de capteur de pression décrit pour chacune des deux chambres de mesures du dispositif.

Il est à noter que, par souci de clarté, les rapports des épaisseurs et dimensions des différents éléments constituant le dispositif n'ont pas été respectés.

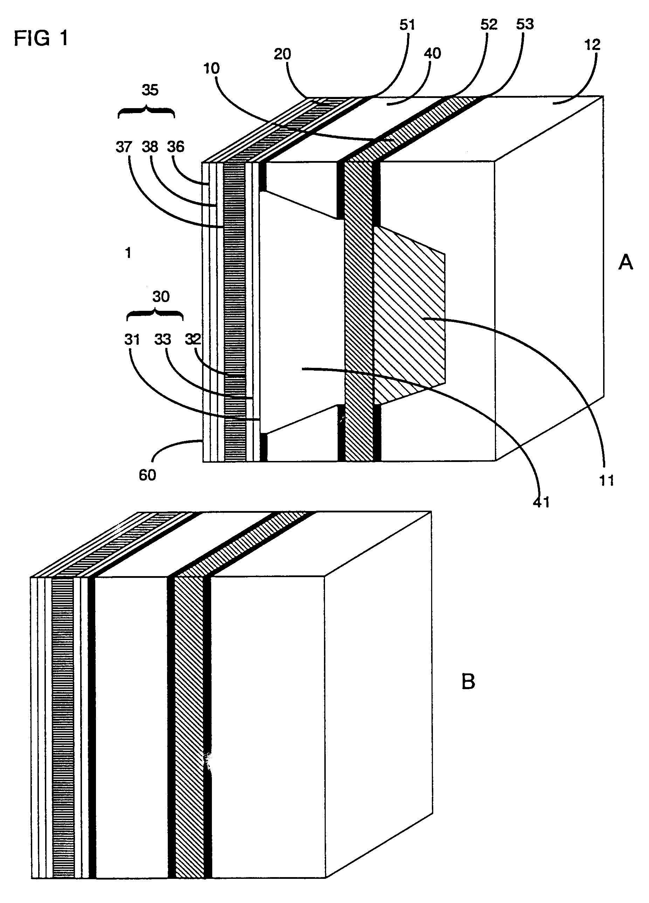

En se référant à la figure 1, on peut décrire les différents éléments constituants l'une des deux chambres de mesures constituant le dispositif selon invention, situé dans un environnement 1 dont on désire mesurer la pression osmotique due aux molécules d'un encombrement équivalent à celui du glucose. Les deux chambres de mesures étant équivalentes du point de vue de leur fonctionnement, nous nous contenterons de décrire la première d'entre elles qui ne se distingue de la seconde que par les propriétés de la membrane hémiperméable utilisée. Pour cette première chambre de mesures, on a fait apparaître sur la figure 1 les deux parties A et B constituant une vision éclatée de cette chambre de mesures selon un plan de coupe sagittal passant par son milieu. La membrane 20 est de nature hémiperméable au glucose, c'est à dire perméable à l'eau et aux molécules plus petites que le glucose, mais imperméable au glucose. L'épaisseur de cette membrane sera choisie en fonction de sa nature. La cavité 41, usinée par procédé photolithographique (avec attaque chimique au KOH) dans une couche de silicium 40 d'environ 30μm d'épaisseur, représente la chambre interne de mesure de la pression osmotique. Le capteur de pression est constitué de la membrane 10 et de la couche 12 ainsi que de la cavité 11, la membrane 10 étant directement en rapport avec la chambre de mesure interne 41. La membrane 10 est une membrane en silicium dopée p + d'environ 20μm d'épaisseur, oxydée sur la région périphérique 53, d'une part, et 52, d'autre part, de façon à être soudée par procédé à chaud (bonding process) à 1000°C à la couche 12, respectivement 40. La couche 53 sert également d'isolant entre la membrane 10 et la couche 12 de façon à constituer un condensateur variable. La couche 12, d'environ 60µm d'épaisseur est creusée en son centre, par procédé photolithographique (avec attaque chimique au KOH), de façon à former une cavité 11 d'environ 20µm d'épaisseur en regard de la couche 10.

Cette cavité 11 doit être parfaitement hermétique pour assurer une bonne précision et une faible dérive du capteur de pression avec le temps.

Le capteur de pression fonctionne, dès lors, comme un condensateur variable dont la capacité varie en fonction des déformations de la membrane 10, sous l'effet de la pression régnant dans la chambre 41.

De façon à optimiser le rendement de mesure de pression au niveau de la membrane 10 du capteur de pression, la membrane 20 est rigidifiée par une couche 30 réalisée, par exemple, en silicium selon la technique décrite par Gjermund Kittilsland et Göran Stemme (Depart. of Solid State Electronics, Chalmer Univ. of Technology, Gothenburg, Suède) et présentée à Montreux, en Suisse, au congrès "Transducer 89", le 26 juin 1989. Un tel type de couche présente l'avantage de pouvoir être réalisée en silicium sur n'importe quelle épaisseur, par conséquent avec un fort coefficient de rigidité, et de disposer de pores de faible diamètre. Ce type de couche est obtenue par soudage de 2 membranes en silicium perforées 31 et 32, les perforations ne se superposant pas et le soudage étant obtenu à chaud (bonding process) grâce à une fine couche d'oxyde de silicium 33 sur les deux surfaces en regards et dont l'épaisseur est parfaitement connue. Cette couche 30 est oxydée en périphérie 51, sur la face en regard de la couche 40, de façon à pouvoir lui être soudée à chaud (bonding process). Une couche de même nature, 35 (constituée de membranes perforées 36 et 37, ainsi que d'une fine couche d'oxyde de silicium 38), peut également être disposée de l'autre côté de la membrane 20.

Afin de ne pas obstruer les pores de la couche de rigidification 30 ou 35, d'une part, ni ceux de la membrane 20, une couche de protection biocompatible 60 vient recouvrir l'ensemble du dispositif. Une telle couche devra, notamment, être perméable aux molécules plus petites que le glucose, ainsi qu'au glucose. Il peut, par exemple, s'agir d'une couche de polymère d'acide perfluoro-sulfonique dont la tenue à long terme est excellente en tissus sous cutané. La protection assurée par cette couche est notamment relative aux cellules et aux dépôts tels que la fibrine.

La deuxième chambre de mesure constituant le dispositif est identique à la première chambre de mesure ainsi décrite à la seule différence de sa membrane 20 qui est choisie perméable limite au glucose, c'est à dire qu'elle laisse passer le glucose mais pas les molécules plus grosses que le glucose.

En définitive la taille d'un tel dispositif, comprenant deux chambres de mesures du type décrit, est tout à fait compatible avec son implantation puisque son épaisseur peut être d'environ 300µm et ses côtés d'environ 2mm.

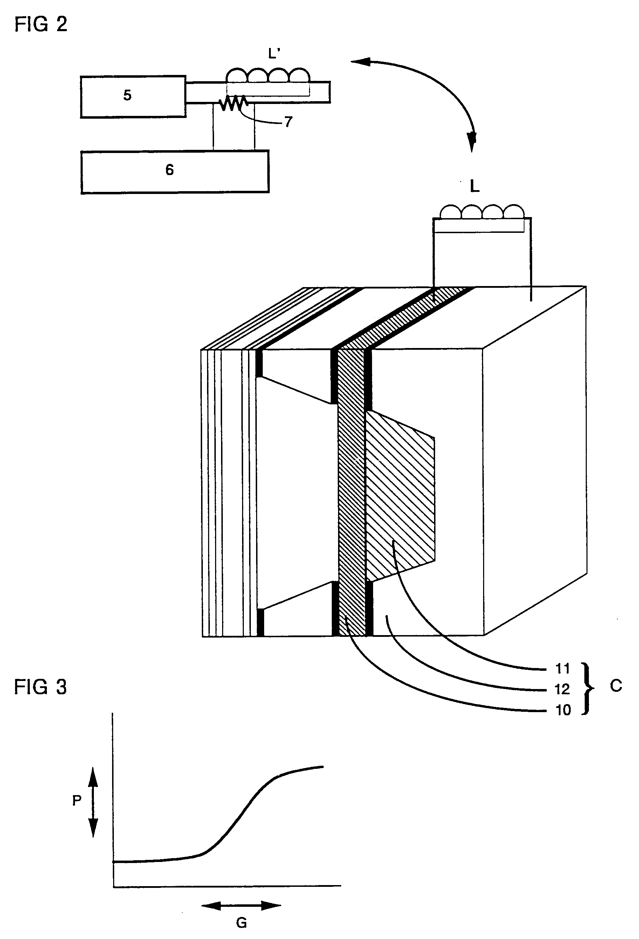

La figure 2 montre le fonctionnement électronique pour chacune des chambres de mesure du dispositif, basé sur le principe d'un circuit résonant passif de type LC. Le condensateur C est obtenu par le capteur de pression constitué des couches 10 et 12, isolées entre elles par la couche d'oxyde de silicium 53. Tout mouvement de la membrane 10, sous l'effet de variations de pression dans la chambre 41, entraîne une modification de la valeur de C. Ce condensateur étant relié en parallèle avec une self L, toute variation de C entraîne automatiquement un changement de la fréquence de résonance d'après la fonction $f = 1/2\pi(LC)^{1/2}$. Cette fréquence de résonance est mesurée à distance par un oscillateur couplé magnétiquement qui convertit la valeur mesurée en pression osmotique dans la cavité 41 et, par différence entre les deux valeurs obtenues au niveau des deux chambres de mesures, en pression osmotique due aux molécules dont l'encombrement est identique à celui du glucose et, par déduction et référence à une valeur de base, en taux de glucose. Cet ensemble électronique externe comprend par conséquent, pour chacune des deux chambres de mesures, un ensemble constitué d'un oscillateur à fréquence variable 5, d'une self L' et d'un détecteur de résonance 6 aux bornes d'une résistance 7, les caractéristiques de résonance pour chacune de ces chambres de mesures étant différentes de façon à pouvoir être analysées depuis l'extérieur sans interférence.

Pour des valeurs courantes de taux de glucose chez un diabétique, pouvant varier entre 0,6 et 3g/l, on peut déduire de la loi de Van't Hoff, relative au calcul de la pression osmotique, les variations de pression osmotique résultantes:

$$\Pi = RT\ c/M$$

ou $\Pi$ = pression osmotique, R = constante des gazs parfaits, T = température absolue en K, c = concentration pondérale, M = masse moléculaire, d'où une variation de la pression osmotique due au glucose entre 60 et 300 mmHg. Pour une mesure précise à quelques pour cents, le capteur de pression devra être, par conséquent, d'une sensibilité de l'ordre de quelques mmHg. Afin de se placer dans les conditions optimales de mesure, fonction des variations physiologiques et des caractéristiques du capteur de pression, on pourra définir une concentration de macro-molécules à placer à l'intérieur de la cavité de mesure de la pression osmotique 41. Pour une large plage de mesure et une grande précision, plusieurs chambres de mesures individuelles pourront être utilisées en parallèles pour simuler une seule chambre de mesure du dispositif, chacune de ces multiples chambres de mesures individuelle disposant dans sa chambre de mesure interne d'une concentration différente de macro-molécules. Bien entendu, les fréquences de résonance de chacun des capteurs seront choisies différentes, de façon à pouvoir analyser chacun des capteurs de mesure individuel depuis l'extérieur sans créer d'interférences.

La figure 3 représente un type de courbe de fréquence de résonance pouvant être obtenue à partir du capteur de pression décrit ci-dessus. On peut voir que le maximum de sensibilité est obtenu pour une certaine plage P de variation de pression qu'il convient d'adapter à la plage utile G de mesure du taux de glucose chez le diabétique.

Afin de limiter l'interaction entre la membrane biocompatible et le milieu environnant, de façon à augmenter la durée de fonctionnement normale du dispositif, on pourra choisir une localisation particulière de l'implant. Ainsi, la cavité péritonéale, le tissus interstitiel sous-cutané sont, de ce point de vue, des régions plus favorables. La région sous cutanée abdominale, par exemple, pourra être particulièrement indiquée étant donné la facilité et innocuité de l'implantation du dispositif en cet endroit.

Bien entendu et afin d'augmenter la sécurité de la mesure effectuée, plusieurs dispositifs pourront être implantés à un même endroit, ou à des endroits différents, le résultat obtenu n'étant pris en compte qu'en présence d'une bonne corrélation entre les mesures effectuées par les différents dispositifs. Dans ce cas, également, les fréquences de résonance de chacun des capteurs individuels seront choisies de façon à ne pas créer d'interférences.

Il convient également de noter que les deux chambres de mesures décrites pour former le dispositif peuvent être réalisées avantageusement dans un même boîtier, voire résulter d'opérations de photolitographie sur un même substrat.

Il est également évident que, selon le type de membrane hémiperméable choisie et, selon sa tenue à long terme, il pourra être nécessaire de mesurer des variations de pression osmotique plutôt que des pressions osmotiques absolues. En effet, seules des membranes totalement imperméable au glucose et exactement imperméable limite au glucose à long terme permettent une mesure absolue de la pression osmotique selon la méthode décrite. Pour des membranes représentants un obstacle plus et moins important au passage du glucose (selon les lois de la diffusion adaptés de Renkin en fonction du diamètre des pores de la membrane), une mesure ne pourra que représenter une différence de pression osmotique par rapport à une valeur de base variable. Aussi, selon la vitesse de passage du glucose pour chacune des membranes, on pourra soit se contenter des caractéristiques spécifiques de ces membranes, soit nécessiter une mesure exacte de la valeur de base à des temps réguliers, par exemple par un prélèvement sanguin.

Un autre intérêt de l'invention est de pouvoir coupler les informations obtenues par le dispositif de mesure directement à une micro-pompe d'insuline, de façon à permettre un ajustement automatique des doses d'insuline à administrer au patient. Un tel système global permettant de constituer un véritable pancréas artificiel.

## Revendications

1. Dispositif implantable d'évaluation du taux de glucose par mesure de la pression osmotique due au glucose comprenant une première chambre de mesure (41) isolée du milieu environnant (1) par une membrane hémiperméable (20) au glucose, cette première chambre étant en rapport avec un premier capteur de pression (10,11,12), lui-même relié à un système électronique (L) permettant d'informer le milieu extérieur à l'organisme de la valeur de la pression mesurée, caractérisé par le fait qu'il comporte une deuxième chambre de mesure isolée du milieu environnant par une membrane perméable au glucose et imperméable aux molécules plus grosses que le glucose, cette deuxième chambre étant en rapport avec un deuxième capteur de pression, lui même relié à un système électronique permettant d'informer le milieu extérieur à l'organisme de la valeur de la pression mesuré, le tout étant agence de telle sorte que l'ensemble des deux capteurs permette, par différence entre les pressions mesurées, d'évaluer de façon précise la pression osmotique due au glucose seul et, par conséquent, le taux de glucose.

2. Dispositif selon la revendication 1, caractérisé en ce que la membrane (20) isolant la chambre de mesure (41) du milieu environnant (1) est rigidifiée par une membrane perforée (30) ayant une faible élasticité, afin de réduire l'atténuation par cette membrane isolante de la pression régnant dans la chambre de mesure et, ainsi, d'optimiser le rendement au niveau du capteur de pression.

3. Dispositif selon la revendication 2, caractérisé en ce que la membrane perforée (30) servant à rigidifier la membrane isolante (20) est réalisée par des procédés photolithographiques.

4. Dispositif selon l'une des revendications 1 à 3, caractérisé en ce que le capteur de pression (10,11,12) est réalisé en silicium par usinage photolithographique.

5. Dispositif selon l'une des revendications 1 à 4, caractérisé en ce que le système électronique relié au capteur de pression est de type passif résonnant LC, dont la fréquence de resonance change en fonction de la pression mesurée, la valeur de cette pression étant déduite par un oscillateur variable couplé magnétiquement et situé à l'extérieur de l'organisme.

6. Dispositif selon l'une des revendications 1 à 5, caractérisé en ce que chacune des chambres de mesures (41) contient des macro-molécules dont le taux est choisi en fonction des valeurs de pression osmotique à mesurer et de la plage de fonctionnnement optimale du capteur de pression.

7. Dispositif selon l'une des revendications 1 à 6, caractérisé en ce que la membrane isolante du milieu environnant est elle-même protégée du milieu dans lequel est implanté le dispositif par une membrane biocompatible (60) imperméable aux cellules, mais perméable au moins au glucose et à l'eau.

8. Procédé pour la mise en oeuvre du dispositif selon l'une des revendications 1 à 7, caractérisé en ce que les résultats des mesures effectuées sont transmis automatiquement à une micro-pompe d'insuline, permettant ainsi d'administrer la quantité requise d'insuline et de substituer l'ensemble à une fonction pancréatique normale du malade.

9. Procédé pour la mise en oeuvre du dispositif selon l'une des revendications 1 à 7, caractérisé en ce que l'interprétation de la mesure de la pression par le capteur, à un instant donné et dans l'une au moins des chambres de mesures, est faite par rapport à ses variations dans le temps et par comparaison à une base de données établie pour une valeur ou des variations connues de taux de glucose sanguin, cette base de données étant éventuellement réétablie à des intervalles de temps fonction de la nature de la membrane hémiperméable choisie ou du patient.

**Claims**

1. An implantable device for estimating the glucose level by measuring the osmotic pressure due to glucose comprising a first measuring chamber (41) insulated from the surrounding medium (1) by a glucose-hemipermeable membrane (20), said first chamber cooperating with a pressure sensor (10,11,12), which in turn is connected to an electronic system (L) for transmitting outside of the body information pertaining to the value of the measured pressure, characterized by the fact that it comprises a second measuring chamber insulated from the surrounding medium by a membrane permeable to glucose and non permeable to molecules larger than glucose, this second chamber cooperating with a second pressure sensor, which in turn is connected to an electronic system for transmitting outside of the body information pertaining to the value of the measured pressure, the whole being arranged so that the assembly of the two sensors provides by difference between the measured pressures an accurate estimation of the osmotic pressure due to the sole glucose and, consequently, of the glucose level.

2. A device according to claim 1, characterized in that the membrane (20) insulating the measuring chamber (41) from the surrounding medium (1) is stiffened by a perforated membrane (30) of a low elasticity, in order to reduce the dampening of the pressure prevailing inside the measuring chamber by this insulating membrane and, therefore, to optimize the yield of the pressure sensor.

3. A device according to claim 2, characterized in that the perforated membrane (30) used for stiffening the insulating membrane (20) is made by photolithographic techniques.

4. A device according to one of claims 1 to 3, characterized in that the pressure sensor (10,11,12) is made of photolithographically machined silicon.

5. A device according to one of claims 1 to 4, characterized in that the electronic system connected to the pressure sensor is a LC-type resonant passive system, the resonnance frequency of which varies as a function of the pressure which is measured, the value of this pressure being determined by a magnetically coupled variable oscillator located outside the body.

6. A device according to one of claims 1 to 5, characterized in that each one of the measuring chambers (41) contains macromolecules, the concentration of which is selected according to the values of the osmotic pressures which are to be measured and to the optimal operational range of the pressure sensor.

7. A device according to one of claims 1 to 6, characterized in that the membrane which provides insulation from the outside medium is in turn protected from the medium in which the device is implanted by a biocompatible membrane (60), which is non permeable to cells, but permeable at least to glucose and to water.

8. Process for the working of the device according to one of claims 1 to 7, characterized in that the results of the measurements carried out are automatically transmitted to an insulin micropump, thus enabling the administration of the required amount of insulin and to use of the assembly as a substitute for a normal pancreatic function of the patient.

9. Process for the working of the device according to one of claims 1 to 7, characterized in that the interpretation of the pressure measured by the sensor, at a given time and at least inside one of the measuring chambers, is made with regards to its variations in the course of time and by comparison with a data base established for a value or for known variations of the blood glucose level, said data base being if required re-established at regular intervals of time of operation according to the nature of the selected hemipermeable membrane or to the patient.

**Patentansprüche**

1. Implantierbare Vorrichtung zur Ermittlung des Glucosegehalts durch Messung des von der Glucose verursachten osmotischen Druckes mit einer durch eine für Glucose halbdurchlässigen Membran (20) vom umgebenden Medium (1) abgeschlossenen ersten Messkammer (41), die mit einem ersten Drucksensor (10, 11, 12) in Beziehung steht, der seinerseits mit einem elektronischen System (L) verbunden ist, das es gestattet, den Wert des gemessenen Druckes in die Körperumgebung zu übermitteln, dadurch gekennzeichnet, dass sie eine zweite Messkammer umfasst, die vom umgebenden Medium durch eine Membran abgeschlossen ist, die für Glucose durchlässig, aber für Moleküle, die glösser als Glucose sind, undurchlässig ist, wobei diese zweite Kammer mit einem zweiten Drucksensor in Beziehung steht, der seinerseits mit einem elektronischen System verbunden ist, das es gestattet, den Wert des gemessenen Druckes in die Körperumgebung zu übermitteln, und wobei das Ganze so eingerichtet ist, dass allein der von der Glucose verursachte osmotische Druck und folglich der Glucosegehalt mit beiden Sensoren zusammen in genauer Weise aus der Differenz zwischen den gemessenen Drücken ermittelt werden kann.

2. Vorrichtung gemäss Anspruch 1, dadurch gekennzeichnet, dass die die Messkammer (41) vom umgebenden Medium (1) abschliessende Membran (20) durch eine perforierte, wenig elastische Membran (30) versteift ist, um die durch diese abschliessende Membran verursachte Abschwächung des in der Messkammer herrschenden Druckes zu vermindern und somit den Wirkungsgrad des Drucksensors zu optimieren.

3. Vorrichtung gemäss Anspruch 2, dadurch gekennzeichnet, dass die zur Versteifung der abschliessenden Membran (20) dienende perforierte Membran (30) durch photolithographische Verfahren geschaffen wird.

4. Vorrichtung gemäss einem der Ansprüche 1 bis 3, dadurch gekennzeichnet, dass der Drucksensor (10, 11, 12) durch photolithographische Bearbeitung aus Silizium geschaffen wird.

5. Vorrichtung gemäss einem der Ansprüche 1 bis 4, dadurch gekennzeichnet, dass das mit dem Drucksensor verbundene elektronische System ein passives LC-Resonanzsystem ist, dessen Resonanzfrequenz sich in Abhängigkeit vom gemessenen Druck ändert, wobei der Wert dieses Druckes durch einen magnetisch gekoppelten, ausserhalb des Körpers befindlichen veränderlichen Schwingungserzeuger abgeleitet wird.

6. Vorrichtung gemäss einem der Ansprüche 1 bis 5, dadurch gekennzeichnet, dass beide Messkammern (41) Makromoleküle enthalten, deren Konzentration in Abhängigkeit von den zu messenden Werten des osmotischen Druckes und vom optimalen Funktionsbereich des Drucksensors gewählt wird.

7. Vorrichtung gemäss einem der Ansprüche 1 bis 6, dadurch gekennzeichnet, dass die vom umgebenden Medium abschliessende Membran selbst durch eine bioverträgliche, für Zellen undurchlässige, aber zumindest für Glucose und Wasser durchlässige Membran (60) vor dem Medium geschützt wird, in das die Vorrichtung implantiert ist.

8. Verfahren für die Anwendung der Vorrichtung gemäss einem der Ansprüche 1 bis 7, dadurch gekennzeichnet, dass die Ergebnisse der ausgeführten Messungen selbsttätig an eine Insulin-Mikropumpe übermittelt werden und es somit erlauben, die erforderliche Menge von Insulin zu verabreichen und das Ganze anstelle der normalen Bauchspeicheldrüsenfunktion des Kranken einzusetzen.

9. Verfahren für die Anwendung der Vorrichtung gemäss einem der Ansprüche 1 bis 7, dadurch gekennzeichnet, dass Sensordruckmessungen zu einem gegebenen Zeitpunkt in zumindest einer der Messkammern im Hinblick auf ihre zeitlichen Veränderungen und im Vergleich zu einer für einen Wert oder bekannte Veränderungen des Glucosegehalts im Blut geschaffene Datenbasis gedeutet werden, wobei diese Datenbasis gegebenenfalls in Zeitabständen aufgefrischt wird, die von der Natur der gewählten halbdurchlässigen Membran oder vom Patienten abhängen.

FIG 1

FIG 2

L'

5

7

6

L

11
12 } C
10

FIG 3

P

G